# EUROPEAN PATENT APPLICATION

(11) **EP 4 678 204 A1**
(43) Date of publication of application: **14.01.2026**
(21) Application number: 23925735.5
(22) Date of filing: 08.03.2023
(51) Int. Cl.: A61M 5/14

(54) **INFUSION DEVICE AND DRIP ASSEMBLY THEREOF**

(71) Applicant: Yu, Gongxin, Taizhou, Zhejiang 31805 (CN)
(72) Inventor: Yu, Gongxin, Taizhou, Zhejiang 31805 (CN)
(74) Representative: Zaboliene, Reda
(86) International application number: PCT/CN2023/080196
(87) International publication number: WO 2024/183012

(57) **Abstract**

The invention provides an infusion set and a drip component thereof, which comprises a drip body, a chamber is arranged inside the drip body, the end of the drip body is divided into a first end and a second end, a liquid outlet is arranged on the drip body, the first end is provided with liquid on-off parts, the liquid on-off part comprises a liquid channel, a first channel opening and a second channel opening are respectively arranged at both ends of the liquid channel, spacing between the second channel opening and the second end in each liquid on-off part is different, the liquid on-off part also comprises a frame body and a liquid level floating body, a liquid inlet hole is arranged on the frame body, and the liquid level floating body is arranged in the frame body and can be blocked or separated from the second channel opening.

## Description

### Technical Field

The invention relates to an infusion set, in particular to an infusion set and a drip component thereof.

### Background Art

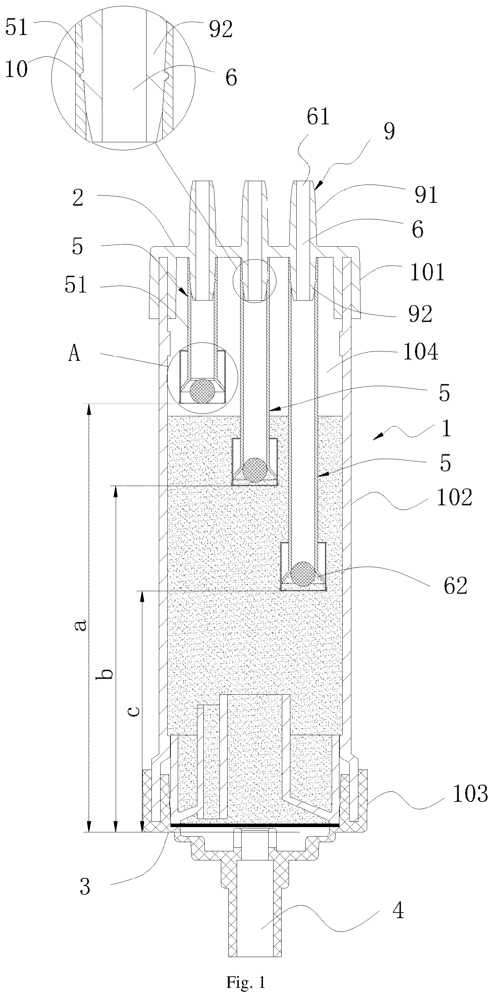

The existing infusion set structure, China Patent CN115591047A, an environmentally friendly disposable infusion set, mainly comprises an infusion bottle, a bottle cap, a puncture device, an infusion tube, a drip and a flow regulator, which are shown in Fig. 1 to the existing patent, the puncture device is inserted into the bottle cap, liquid in the infusion bottle passes through the bottle cap, the puncture device, the infusion tube, the drip and the flow regulator in sequence, and finally flows into the body of the patient.

Some patients use more than one infusion bottle in the infusion process, and the liquid in the infusion bottles needs to be injected into the patient one by one.

During the actual operation, the medical staff first hangs several infusion bottles above the infusion support at one time, and inserts the puncture device into one infusion bottle. After the infusion of liquid in the infusion bottle is completed, the medical staff pulls the puncture device out of the infusion bottle, and then inserts the puncture device into another infusion bottle to continue infusion.

During the above infusion process, the patient needs to always pay attention to the total amount of liquid residue in the current infusion bottle. When the total amount of liquid residue in the infusion bottle is small, the patient only needs to call the medical staff to pull the puncture device out of the infusion bottle and insert the puncture device into another infusion bottle.

Based on the realistic medical care environment, on the one hand, the physical state of the patient is so poor that the patient cannot always keep an eye on the total amount of liquid residue in the current infusion bottle, and the companion cannot always pay attention to the total amount of liquid residue even if the patient is accompanied; on the other hand, the number of medical staff is limited, and the medical staff cannot always pay attention to the situation of each infusion patient; once the infusion of liquid in the infusion bottle is completed and the infusion bottle is not changed in time, the blood of the patient is likely to flow back to the infusion tube, and even air accidentally enters the blood vessel of the patient, which is easy to cause medical accidents.

### Summary of the Invention

In view of this, the first purpose of the invention is to provide a drip component, which automatically triggers the liquid level floating body in the liquid on-off part to float up and down by taking advantage of the water level in the chamber, and the corresponding second opening can be automatically opened or closed, thereby finally realizing the function of automatically switching infusion bottles and effectively reducing the probability of medical accidents.

In order to solve the above technical problems, the invention adopts the following technical proposal:
a drip component comprises a drip body, a chamber is arranged inside the drip body, two opposite ends of the drip body are respectively a first end and a second end, and a liquid outlet is arranged on the drip body;
the first end is provided with two or more liquid on-off parts;
the liquid on-off part comprises a liquid channel, a first channel opening and a second channel opening are arranged at both ends of the liquid channel, the first channel opening extends to the outside of the drip body, the second channel opening is located in the chamber and extends in the direction of the second end, and spacing between the second channel opening and the second end in each liquid on-off part is different;
the liquid on-off part also comprises a frame body and a liquid level floating body, the frame body is installed on the outside of the second channel opening, the frame body is provided with a liquid inlet hole, and the liquid level floating body is arranged in the frame body and can be blocked or separated from the second channel opening.

Through the above technical proposal, in the actual use process, the liquid first flows into the chamber from the second channel farthest from the second end until the liquid level in the chamber is higher than the second channel opening of the other liquid on-off part; at this time, the liquid in the chamber pushes the liquid level floating body in the other liquid on-off part, the liquid level floating body is blocked in the corresponding second channel opening, and other liquid cannot flow into the chamber through the liquid on-off part blocked by the liquid level floating body.

The liquid in the second channel opening farthest from the second end continuously flows into the chamber; when the liquid in the second channel opening farthest from the second end drains away, the liquid level in the chamber gradually decreases until the liquid level is reduced to the position below the second channel opening of the other liquid on-off part, the corresponding liquid level floating body is separated from the second channel opening, and then the liquid in the corresponding liquid channel can gradually flow into the chamber.

In the above use process, the difference of liquid level in the cavity is fully utilized, thereby realizing the functions of ensuring different liquids automatically flow into the chamber and automatically switching infusion bottles, abandoning the disadvantage that the medical staff needs to remove the needle and change the bottle in the traditional infusion process, alleviating the need of the patient to pay attention to the amount of liquid in the infusion bottle, and effectively reducing the probability of medical accidents.

Preferably, a mounting hole is arranged on the frame body, the second channel opening is extended into the frame body from the mounting hole and is in a shape of flaring, and the flaring of the second channel opening faces towards one side of the liquid level floating body.

Through the above technical proposal, the liquid level floating body more easily enters the second channel opening in a flaring shape, and the sealing accuracy is higher, and the effect is better.

Preferably, the flaring size of the second channel opening is larger than the pore size of the mounting hole on the frame body.

Through the above technical proposal, the flaring of the second channel opening can be matched with the mounting hole of the frame body to improve the frame body .

Preferably, the flaring outer edge of the second channel opening is butted against the inner frame wall of the frame body.

Through the above technical proposal, collision force is generated between the flaring outer edge of the second channel opening and the inner frame wall of the frame body, which helps improve the installation stability between the frame body and the second channel opening, so that the floating space of the liquid level floating body can be controlled, and the sealing or dredging of the second channel opening is more accurate.

Preferably, the frame body comprises a frame and a frame cover, the mounting hole is located on the frame, the liquid inlet hole is located on the frame cover, a frame port in the same direction as the second channel opening is arranged on the frame, and the frame cover is removably connected to the frame port of the frame.

Through the above technical proposal, in the actual installation process, the liquid level floating body can be first placed into the frame, and then the frame cover is installed on the frame, so that the installation is convenient.

Preferably, the drip body comprises a first drip cap and a main drip body, the first end is located on the first drip cap, and the end part of the main drip body is inserted on the side wall of the first drip cap.

Through the above technical proposal, the first drip cap and the main drip body are arranged separately; on the one hand, the separate design is adopted to reduce the difficulty of manufacturing; on the other hand, in the actual installation process, the liquid on-off part can be installed on the first drip cap, and then the first drip cap can be installed at the end of the main drip body, which makes the installation process more convenient.

Preferably, two or more butting pieces are arranged on the first drip cap, each butting piece comprises an outer butt joint and an inner butt joint, the outer butt joints extend to the outside of the drip body, the inner butt joints extend to the inside of the chamber, and the liquid channel is located in the outer butt joint (91), the first drip cap and the inner butt joint.

The liquid on-off part comprises a liquid on-off tube, the end of the liquid on-off tube is inserted into the inner butt joint, and the second channel opening is located at the end of the liquid on-off tube deviated from the inner butt joint.

Through the above technical proposal, firstly, the butting piece and the first drip cap can be integrally molded by injection, which effectively reduces the manufacturing cost; secondly, the separate design is adopted between the liquid on-off tube and the inner butt joint, which is convenient for disassembly and assembly; thirdly, combined with the separate design between the first drip cap and the main drip body, the liquid on-off tube can be first inserted into the inner butt joint on the first drip cap, and then the first drip cap is installed at the end of the main drip body, which has high installation stability and can ensure sufficient installation convenience.

Preferably, the drip body also comprises a second drip cap, the end of the main drip body deviated from the first drip cap is inserted on the side wall of the second drip cap, and the liquid outlet is located on the second drip cap.

Through the above technical proposal, the first drip cap, the main drip body and the second drip cap in the whole drip body adopt split design, which reduces the difficulty of production and is convenient to install.

Preferably, a clamping ring is arranged on the outer side wall of the inner butt joint, and the liquid on-off tube is clamped on the clamping ring.

Through the above technical proposal, the clamping ring of the inner butt joint is buckled with the inner wall of the liquid on-off tube when the liquid on-off tube is inserted into the inner butt joint, so that the connection between the liquid on-off tube and the inner butt joint is more stable.

Preferably, a plug is arranged on the liquid level floating body, extends to the second channel opening, and can be blocked or separated from the second channel opening.

Through the above technical proposal, The liquid level floating body is placed in the liquid environment, the liquid exerts upward buoyancy effect on the liquid level floating body with the continuous rise of the liquid level, and the liquid level floating body pushes the plug into the second channel opening to produce a blocking effect; of course, the liquid level floating body is also butted with the second channel opening to produce a certain blocking effect; at this time, the liquid in the liquid channel cannot flow into the liquid environment smoothly; on the contrary, when the liquid level of the liquid environment continues to fall, the liquid level floating body also moves downward, and the plug on the liquid level floating body is gradually removed from the second channel opening; at this time, the liquid in the liquid channel can flow into the liquid environment smoothly.

A liquid level floating sensing device of the above structure can use a small volume liquid level floating body to push the plug to move, not only the liquid plugging and sealing effect is outstanding, but also the volume of the liquid level floating body can be effectively controlled, and the volume of the entire device can also be effectively controlled.

Preferably, a sealing groove is arranged on the inner frame wall of the frame body and is located on one side close to the second channel opening.

When the plug is blocked in the second channel opening, the side wall of the liquid level floating body is butted in the sealing groove.

Through the above technical proposal, the outer side wall of the liquid level floating body is butted in the sealing groove when the plug is blocked in the second channel opening, and the sealing relationship is formed between the liquid level floating body and the sealing groove to greatly improve the sealing effect of the liquid level floating body, the plug, the second channel opening and the sealing groove.

Preferably, a guide head is arranged on the liquid level floating body, a guide hole is arranged on the frame body, and the guide head is connected to the guide hole in a slipping way.

Through the above technical proposal, the guide head and the guide hole are mutually matched when the liquid level floating body floats up and down, and the plug can also be matched with the second channel opening, so that the floating stability of the whole liquid level floating body is higher.

Preferably, the frame body comprises a frame and a frame cover, the frame cover is removably connected to the frame, and the guide hole is located on the frame cover.

Through the above technical proposal, the frame cover can be opened from the frame, and then the liquid level floating body can be installed into the frame, so that the installation between the liquid level floating body and the frame is more convenient.

Preferably, a guide groove is arranged on the inner frame wall of the frame body, and mutually slip fit is adopted between the side wall of the liquid level floating body and the guide groove.

Through the above technical proposal, the guide head and the guide hole are mutually matched when the liquid level floating body floats up and down, the plug can also be matched with the second channel opening, and the slip fit is adopted between the side wall of the liquid level floating body and the guide groove, so that the floating stability of the whole liquid level floating body is further improved.

Preferably, the liquid on-off part comprises a liquid on-off tube, which is integrally molded on the frame body by injection.

Through the above technical proposal, not only the production efficiency of the whole device is greatly improved, but also the structural stability is effectively promoted.

Preferably, the liquid level floating body comprises a first floating body and a second floating body, and the plug is installed on the first floating body, and the first floating body and the second floating body are buckled mutually.

Through the above technical proposal, the structural complexity of a single injection mold is reduced, and the later installation is relatively more convenient.

Preferably, a cavity is formed between the first floating body and the second floating body buckled mutually.

Through the above technical proposal, on the one hand, materials are saved; on the other hand, the liquid level floating body with the cavity has greater buoyancy and higher sensitivity to float up and down.

The second purpose of the invention is to provide an infusion set, the drip component in the infusion set can automatically trigger the liquid level floating body in the liquid on-off part to float up and down by taking advantage of the water level in the chamber, the corresponding second channel opening can be automatically opened or closed, and the whole infusion set can finally realize the function of automatically switching infusion bottles and effectively reduces the probability of medical accidents.

In order to solve the above technical problems, the invention adopts the following technical proposal:
an infusion set comprises an infusion bottle, a puncture device, an infusion tube a, an infusion tube b, a flow regulator and an infusion needle, and also comprises the drip component;
at least two infusion bottles, two puncture devices and two infusion tubes a are arranged, the infusion bottles, the puncture devices and the infusion tubes a correspond to the butting pieces one by one, the puncture devices are inserted into the corresponding infusion bottles, the infusion tube a is connected between the puncture device and the outer butt joint, the infusion tube b is connected between the liquid outlet and the infusion needle, and the flow regulator is installed on the infusion tube b.

Through the above technical proposal, the puncture device corresponding to the liquid on-off part furthest from the second end of the second channel opening is first inserted into the infusion bottle, then the liquid in the infusion bottle flows into the chamber until the liquid level in the chamber is located below the second channel opening of the liquid on-off part furthest from the second end, the liquid on-off part furthest from the second end is recorded as an on-off part A, and the liquid level in the chamber is higher than the second channels of all liquid on-off parts except the on-off part A; at this time, other puncture devices can be inserted into other infusion bottles; during the infusion process, when the liquid in the first infusion bottle is dripped, the liquid level in the chamber drops and is directly lower than the second channel openings of other liquid on-off parts, and the liquid in the second infusion bottle can drop directly, thereby realizing the function of automatically switching infusion bottles and effectively reducing the probability of medical accidents.

### Brief Description of the Drawings

Fig. 1 is the structural diagram of embodiment 1.
Fig. 2 is the enlarged view for Part A of Fig. 1.
Fig. 3 is the schematic diagram of structural changes in embodiment 1 during the liquid level drop.
Fig. 4 is the structural diagram of embodiment 2.
Fig. 5 is the structural diagram of embodiment 4.
Fig. 6 is the enlarged view for Part B of Fig. 5.
Fig. 7 is the structural diagram of the partition in embodiment 4.
Fig. 8 is the local section view of the partition in embodiment 4.

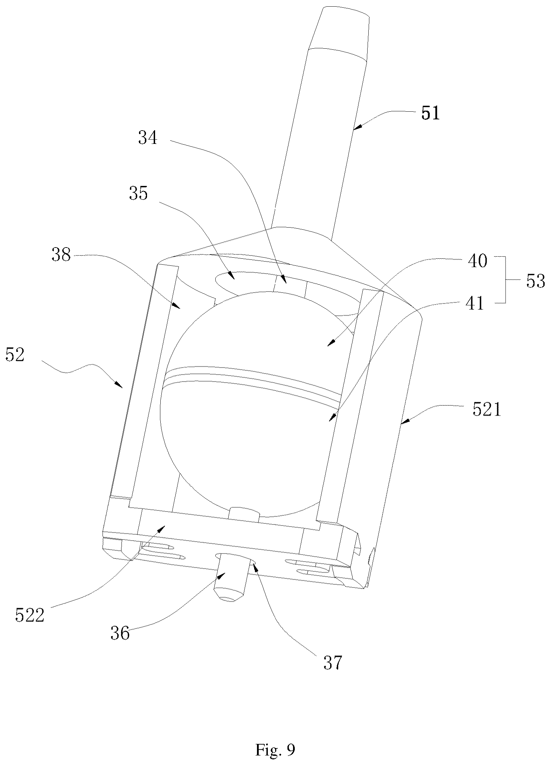

Fig. 9 is the structural diagram of the liquid on-off part in embodiment 6.

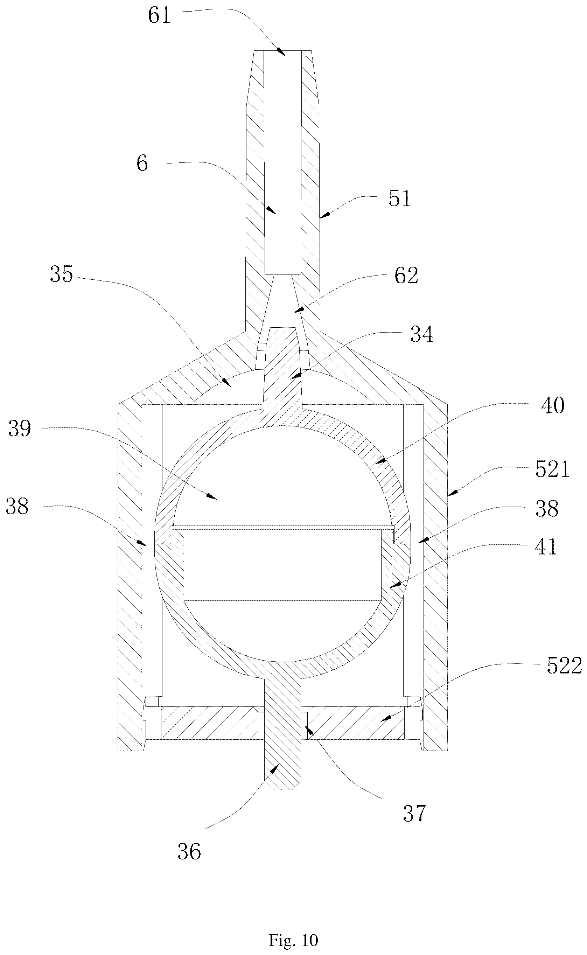

Fig. 10 is the sectional structure diagram of the liquid on-off part in embodiment 6.

Reference signs: 1. Drip body; 101. First drip cap; 102. Main drip body; 103. Second drip cap; 104. Chamber; 2. First end; 3. Second end; 4. Liquid outlet; 5. Liquid on-off part; 51. Liquid on-off tube; 52. Frame body; 521. Frame; 522. Frame cover; 53. Liquid level floating body; 6. Liquid channel; 61. First channel opening; 62. Second channel opening; 7. Liquid inlet hole; 8. Mounting hole; 9. Butting piece; 91. Outer butt joint; 92. Inner butt joint; 10. Clamping ring; 11. Infusion bottle; 12. Puncture device; 13. Infusion tube A; 14. Infusion tube B; 15. Flow regulator; 16. Infusion needle; 17. Frame port; 18. Control valve; 19. Partition; 20. Tube through hole; 21. Drive rod; 22. Elastic protective sleeve; 23. Drive auxiliary rod; 24. Extension tube section; 25. Pressing hole; 26. Pressing piece; 27. Slot; 28. Interval space; 29. Seal ring; 30. Elastic part; 31. Butting block; 32. Butting slot; 33. Drive hole; 34. Plug; 35. Sealing groove; 36. Guide head; 37. Guide hole; 38. Guide groove; 39. Cavity; 40. First floating body; 41. Second floating body.

### Detailed Description of the Invention

The embodiments of the invention are further detailed in combination with the drawings, so that the technical proposal of the invention is more easily understood and mastered.

### Embodiment 1:

A drip component, as shown in Fig. 1, triggers the structure inside different liquid on-off parts 5 by using the change of liquid level height in the chamber 104 and open or close the channels in the corresponding liquid on-off parts 5, realizing the purpose of automatic step-by-step liquid flowing.

As shown in Fig. 1, the drip component structure comprises a drip body 1, and the two opposite ends of the drip body 1 are respectively the first end 2 and the second end 3; by taking Fig. 1 as the reference orientation, the first end 2 is located at the top and the second end 3 is located at the bottom, which are the same as the reference orientation in actual use.

As shown in Fig. 1, the drip body 1 comprises a first drip cap 101, a main drip body 102 and a second drip cap 103.

As shown in Fig. 1, the first end 2 is located on the first drip cap 101, and the upper end of the main drip body 102 is inserted on the side wall of the first drip cap 101; the second end 3 is located on the second drip cap 103, and the lower end of the main drip body 102 is inserted into the side wall of the second drip cap 103.

A liquid outlet 4 is arranged on the second drip cap 103.

A chamber 104 is arranged inside the drip body 1.

The drip component also comprises two or more liquid on-off parts 5. The embodiment is described in detail by arranging three liquid on-off parts 5.

The liquid on-off parts 5 are located on the first end 2.

Two or more butting pieces 9 are arranged on the first drip cap 101, and the butting pieces 9 correspond to the liquid on-off parts 5 one to one. Similarly, the arrangement of three butting pieces 9 is also taken as an example. Each of the butting pieces 9 comprises an outer butt joint 91 and an inner butt joint 92, the outer butt joint 91 extends to the outer side of the drip body 1, and the inner butt joint 92 extends to the inner side of the chamber 104. The liquid channel 6 is arranged in the outer butt joint 91, the first drip cap 101 and the inner butt joint 92, the first channel opening 61 and the second channel opening 62 are respectively arranged at both ends of the liquid channel 6, the first channel opening 61 extends to the outer side of the drip body 1, the second channel opening 62 is located in the chamber 104 and extends in the direction of the second end 3, that is, the two channel openings of the liquid channel 6 respectively lead to the outside of the drip body 1 and the chamber 104.

Each liquid on-off part 5 comprises a liquid on-off tube 51, the end of the liquid on-off tube 51 is inserted into the inner butt joint 92, and the second channel opening 62 is located at the end of the liquid on-off tube 51 away from the inner butt joint 92.

The spacing between the second channel opening 62 and the second end 3 in each liquid on-off part 5 is different. In detail, as shown in Fig. 1, the liquid on-off tube 51 is a straight tube, the length direction of the liquid on-off tube 51 is vertical, the distance between the second channel opening 62 at the lower end of the left liquid on-off tube 51 and the second end 3 is a, the distance between the second channel opening 62 at the lower end of the middle liquid on-off tube 51 and the second end 3 is b, the distance between the second channel opening 62 at the lower end of the right liquid on-off tube 51 and the second end 3 is c, wherein a is greater than b, and b is greater than c.

To improve the plug stability between the liquid on-off tube 51 and the inner butt joint 92, a clamping ring 10 is arranged on the outer side wall of the inner butt joint 92, and the liquid on-off tube 51 is clamped on the clamping ring 10.

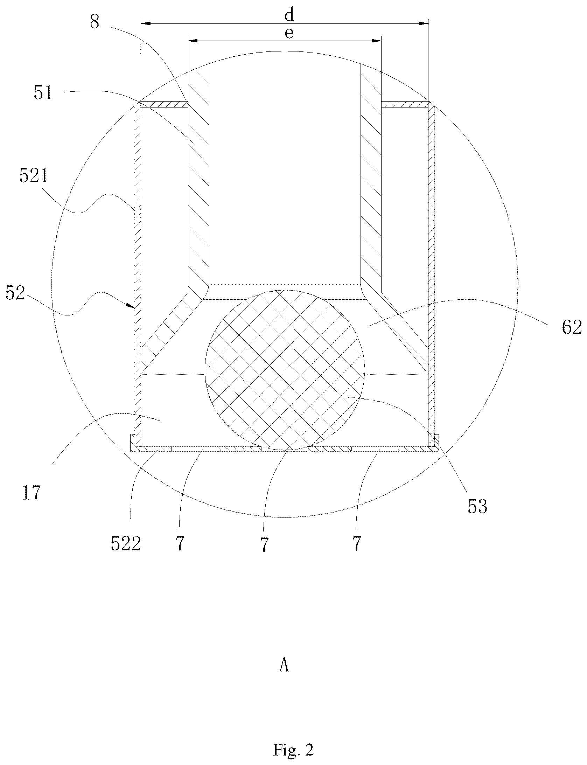

As shown in Fig. 1 and Fig. 2, the liquid on-off part 5 also comprises a frame body 52 and a liquid level floating body 53, the frame body 52 is installed on the outside of the second channel opening 62, a liquid inlet hole 7 is provided on the frame body 52, and the liquid level floating body 53 is arranged in the frame body 52. A certain space is formed between the frame body 52 and the second channel opening 62, providing a certain floating distance to the liquid level floating body 53. If the liquid level floating body 53 floats upwards due to the liquid buoyancy in the chamber 104, the liquid level floating body 53 can be blocked at the opening part of the second channel opening 62, and the second channel opening 62 can be blocked; at this time, the liquid in the liquid on-off tube 51 cannot flow into the chamber 104. Conversely, if the liquid level floating body 53 is not affected by the liquid buoyancy in the chamber 104, the liquid level floating body 53 falls under the action of gravity and is then separated from the second channel opening 62, and the second channel opening 62 can be opened; at this time, the liquid in the liquid on-off tube 51 can flow into the chamber 104.

To improve the floating sensitivity of the liquid level floating body 53, the liquid level floating body 53 can also be hollow.

A mounting hole 8 is arranged at the position where the frame body 52 is close to the first end. The second channel opening 62 extends into the frame body 52 through the mounting hole 8. The second channel opening 62 is in a flaring shape, and the flaring of the second channel opening 62 faces towards the side of the liquid level floating body 53.

As shown in Fig. 2, the flaring size of the second channel opening 62 is d, and the pore size of the mounting hole 8 on the frame body 52 is e, wherein d is larger than e. The flaring outer edge of the second channel opening 62 is butted against the inner frame wall of the frame body 52.

The frame body 52 also comprises a frame cover 522, and the liquid inlet hole 7 is located on the frame cover 522.

The frame port 17 in the same direction as the second channel opening 62 is arranged on the frame 521, and the frame cover 522 is removably connected to the frame port 17 of the frame 521. The frame cover 522 and the frame port 17 of the frame 521 shown in the embodiment are connected by cover closing.

### Embodiment 2:

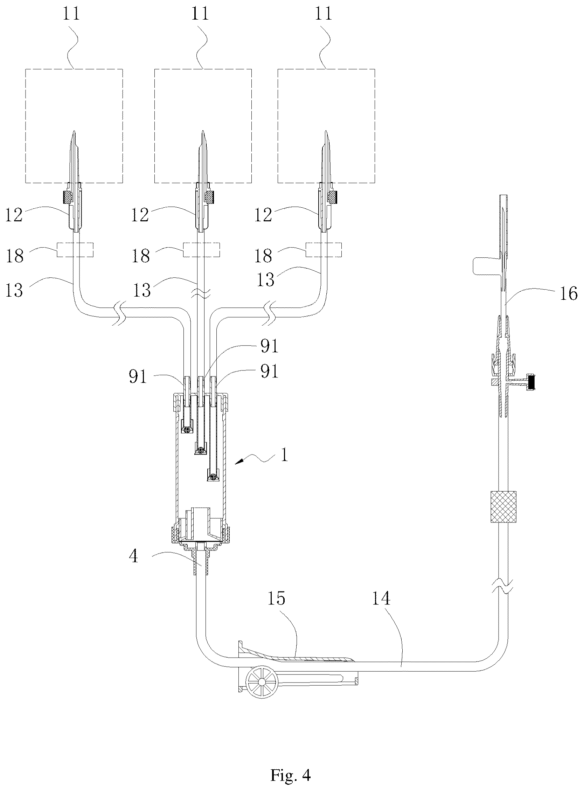

An infusion set, as shown in Fig. 4, comprises an infusion bottle 11, a puncture device 12, an infusion tube A 13, an infusion tube B 14, a flow regulator 15, an infusion needle 16 and the drip component in embodiment 1.

At least two infusion bottles 11, two puncture devices 12 and two infusion tubes A 13 are arranged. The quantities of infusion bottles 11, puncture devices 12 and infusion tubes 13 shown in the embodiment are three respectively.

The infusion bottles 11, the puncture devices 12 and the infusion tubes A 13 correspond to the butting pieces 9 one by one, the puncture devices 12 are inserted into the corresponding infusion bottles 11, the infusion tube A 13 is connected between the puncture device 12 and the outer butt joint 91, the infusion tube B 14 is connected between the liquid outlet 4 and the infusion needle 16, and the flow regulator 15 is installed on the infusion tube B 14.

In addition, to further improve the ease of operation, each infusion tube A 13 can also be equipped with a control valve 18, which can be used to control the liquid on-off of the infusion tube 13.

### Embodiment 3:

Based on the infusion set structure in the embodiment 2, the specific use steps of the infusion set are as follows:
step 1: mark the three puncture devices 12 as the first puncture device, the second puncture device and the third puncture device; the distances between the second channel openings 62 at the lower ends of the liquid on-off tubes 51 corresponding to the first puncture device, the second puncture device and the third puncture device and the second end 3 are from far to near in sequence;
   the control valves 18 corresponding to the first puncture device, the second puncture device and the third puncture device are the first valve, the second valve and the third valve respectively;
   insert the first puncture device, the second puncture device and the third puncture device into the corresponding infusion bottles 11 respectively;
step 2: open the first valve, close the second valve and the third valve, and turn on the flow regulator 15;
step 3: after the liquid level in the chamber 104 is higher than the second channel opening 62 corresponding to the second puncture device and the third channel opening corresponding to the third puncture device, close the first valve and turn off the flow adjuster 15;
step 4: insert the infusion needle 16 into the vein of the patient;
step 5: turn on the flow regulator 15, and open the first valve, the second valve and the third valve;
step 6: turn off the flow regulator 15 after the injection of all infusion bottles 11 is completed.

Under the above use steps, even if there are multiple infusion bottles 11, automatic bottle-by-bottle infusion effect can be achieved.

After the operation of Step 5, the flow of the liquid inside the infusion set is as follows:
as shown in fig. 1, the liquid in the infusion bottle 11 corresponding to the first puncture device flows into the chamber 104 of the main drip body 102 through the liquid on-off tube 51 located on the far left, and the liquid in the chamber 104 continuously flows into the infusion tube b 14 and the infusion needle 16 from the liquid outlet 4; at this time, the state presented is shown in fig. 1.

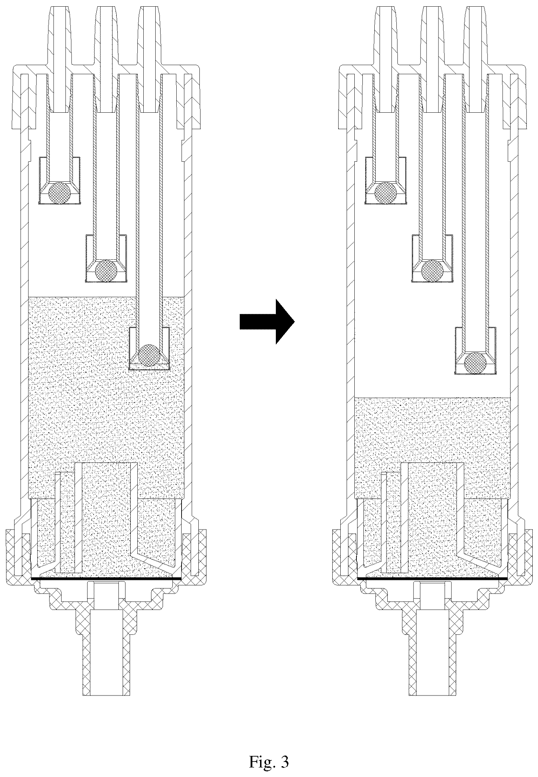

When the liquid in the infusion bottle 11 corresponding to the first puncture device finishes dripping, the liquid level in the chamber 104 of the main drip body 102 drops accordingly; at this time, the liquid level floating body 53 of the liquid on-off tube 51 corresponding to the second puncture device moves downward, the second channel opening 62 at the lower end of the liquid on-off tube 51 is opened, and the liquid in the corresponding infusion bottle 11 continuously flows into the chamber 104 through the second channel opening 62; at this time, the state presented is shown in the left schematic diagram of Fig. 3.

When the liquid in the infusion bottle 11 corresponding to the second puncture device finishes dripping, the liquid level in the chamber 104 of the main drip body 102 continues to drop; at this time, the liquid level floating body 53 of the liquid on-off tube 51 corresponding to the third puncture device moves downward, the second channel opening 62 at the lower end of the liquid on-off tube 51 is opened, and the liquid in the corresponding infusion bottle 11 continuously flows into the chamber 104 through the second channel opening 62; at this time, the state presented is shown in the right schematic diagram of Fig. 3.

### Embodiment 4:

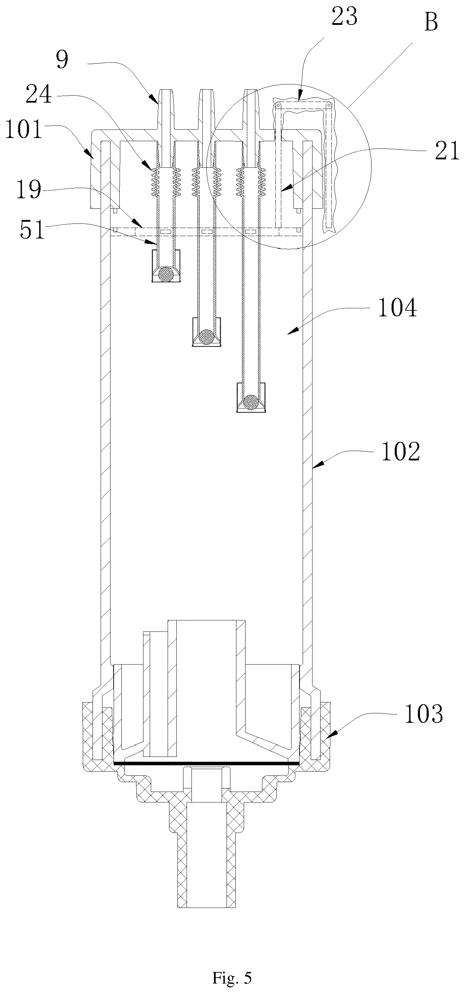

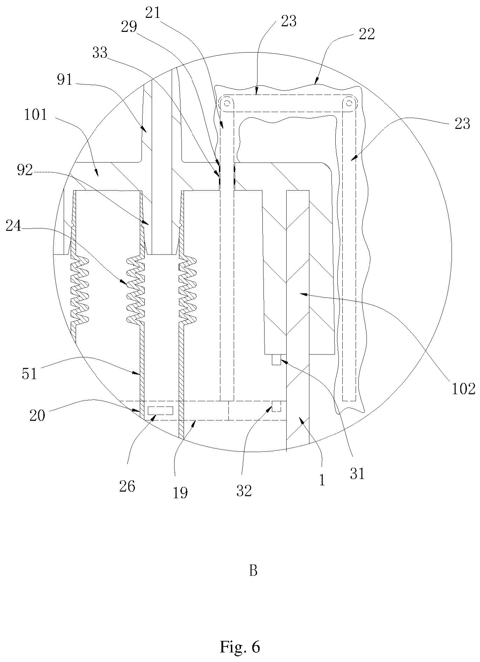

Based on the structural basis of the embodiment 1 or embodiment 2, the following structures are added:
As shown in Fig. 5 and Fig. 6, a partition plate 19 is arranged in the chamber 104, the outer edge of the partition plate 19 is butted with the inner wall of the chamber 104, and a tube through hole 20 through which a liquid on-off tube 51 passes is arranged on the partition plate 19.

Therefore, in the clinical operation process, on the one hand, to make the liquid in infusion bottle 11 flow into the chamber 104 more quickly, the medical staff usually hold the main drip body 102 before infusion, so that the liquid in the infusion bottle 11 can quickly flow into the chamber 104 during the deformation recovery of the chamber 104 in the main drip body 102 from small to large; on the other hand, the deformation range of the main drip body 102 also directly determines the amount of liquid entering the chamber 104, that is, the liquid level in the chamber 104.

Based on the above operating principle, the partition 19 is arranged so that the medical staff only need to hold the main drip body 102 located in the lower part of the partition 19, and the main drip body 102 located in the upper part of the partition 19 is not deformed, that is, the partition 19 can control the maximum deformation amount of the main drip body 102; the medical staff only need to squeeze the main drip body 102 located at the lower part of the partition 19 to minimize the problem of height difference in liquid level in the chamber 104 caused by the different hand strength of the medical staff.

Next, the partition 19 with the tube through hole 20 has the position fixing effect on the liquid on-off tube 51 to minimize the change of the distance between the second channel opening 62 and the second end 3 caused by the shaking of the main drip body 102 and the accidental tilt of the liquid on-off tube 51 during the infusion process, and the infusion stability is higher.

As shown in Fig. 5 and Fig. 6, the partition 19 is connected with a drive rod 21, a drive hole 33 is arranged on the first drip cap 101, the drive rod 21 passes through the drive hole 33 and extends to the outside of the drip body 1 and can push the partition 19 to move along the chamber 104, a seal ring 29 is installed on the hole wall of the drive hole 33, and the drive hole 33 and the drive rod 21 are butt-sealed through the seal ring 29.

The drip component also comprises an elastic protective sleeve 22, and the end of the elastic protective sleeve 22 is sealed at the opening of the drive hole 33, and the end of the drive rod 21 located outside the drip body 1 is located inside the elastic protective sleeve 22.

Therefore, firstly, when needing to drive the partition 19 to move up and down, the medical staff can apply force to the drive rod 21, which can drive the partition 19 to move up and down and then control the maximum deformation amount generated by the main drip body 102 in use state; secondly, the seal ring 29 can maintain the airtightness inside the chamber 104; thirdly, while ensuring the flexible movement of the drive rod 21, the elastic protective sleeve 22 can block the drive rod 21 from the outside to prevent external bacteria from entering the chamber 104.

As shown in Fig. 6, at least one drive auxiliary rod 23 is hinged at the end of the drive rod 21 located outside the drip body 1, and the drive auxiliary rod 23 is located in the elastic protective sleeve 22.

Therefore, the drive auxiliary rod 23 and the drive rod 21 are hinged to each other, so that the moving distance of the partition 19 is ensured, and relative rotation can occur between the drive auxiliary rod 23 and the drive rod 21 when the partition 19 is not required to be driven for movement, and the space occupancy rate is low.

As shown in Fig. 6, the number of drive auxiliary rods 23 is two, and the drive auxiliary rods 23 are hinged with the drive rod 21 and can be fitted on the outer cap wall of the first drip cap 101.

Therefore, when the partition 19 is not required to be driven for movement, the drive auxiliary rod 23 and the drive rod 21 can rotate each other and can be fitted to the outer cap wall of the first drop cap 101, minimizing the space occupancy and reducing the possibility of accidental touch and push.

As shown in Fig. 6, the liquid on-off tube 51 comprises a telescopic tube section 24, which can be pulled by external force to produce length changes;a pressing hole 25 is arranged on the partition 19 and corresponds to the tube through hole 20 one to one, a pressing piece 26 is installed in the pressing hole 25, one end of the pressing piece 26 faces toward the liquid on-off tube 51 in the tube through hole 20, and the other end of the pressing piece 26 faces toward the inner wall of the chamber 104.

After the outer side wall of the main drip body 102 is deformed toward the inside of the chamber 104, the outer side wall of the main drip body 102 acts on the end of the pressing piece 26, which can be pressed against the outer tube wall of the liquid on-off tube 51.

Therefore, in the medical care scenario, saline and albumin need to be injected intravenously into some patients; during the practical operation, saline is stored in one infusion bottle 11, and albumin is stored in another infusion bottle 11. Different from ordinary infusion, such infusion includes the following steps: Step 1: Inject part of the first bottle of normal saline into the patient; Step 2: Inject the second bottle of albumin into the patient; Step 3: Inject the remaining part of the first bottle of normal saline into the patient again. Therefore, corresponding to the above three steps, the medical staff need to insert the puncture device 12 into the infusion bottle 11 containing normal saline, then pull out and insert the puncture device 12 into the infusion bottle 11 containing albumin and finally pull out and insert the puncture device 12 into the infusion bottle 11 containing normal saline. In the above operation process, two problems are involved: first, the puncture device 12 is inserted and pulled out twice, easily causing pollution; second, the medical staff perform at least two operations, being inconvenient.

Even for such special use scenarios and operation problems as above, in the application, two puncture devices 12 are first inserted into the infusion bottle containing normal saline 11 and the infusion bottle containing albumin 11, the second channel opening 62 of the liquid on-off part 5 corresponding to the infusion bottle 11 containing normal saline is farthest from the second end 3, and the second channel opening 62 of the liquid on-off part 5 corresponding to the infusion bottle 11 containing albumin is closest to the second end 3.

First, part of the normal saline in the infusion bottle 11 is injected into the patient; when a specified amount of normal saline is injected, the medical staff can hold the outer side wall of the main drip body 102, the outer side wall of the main drip body 102 is deformed inward, the wall of the chamber 104 pushes the end of the pressing piece 26, the pressing piece 26 moves to the side of the tube through hole 20, and the end of the pressing piece 26 is butted with the liquid on-off tube 51 corresponding to the normal saline infusion bottle 11 in the tube through hole 20; the medical staff exert force on the drive auxiliary rod 23 and the drive rod 21, and the whole partition 19 moves downward and drives the liquid on-off tube 51 corresponding to the normal saline infusion bottle 11 to move downward, the elastic protective sleeve 22 is stretched, the second channel opening 62 of the liquid on-off tube 51 is located below the liquid level of the chamber 104, and the liquid level floating body 53 is blocked in the second channel opening 62, and the normal saline cannot continue to flow into the chamber 104.

In the same operation method, the medical staff hold the outer side wall of the main drip body 102, the outer side wall of the main drip body 102 is deformed inward, the wall of the chamber 104 pushes the end of another pressing piece 26, the pressing piece 26 moves to the side of the tube through hole 20, and the end of the pressing piece 26 is butted with the liquid on-off tube 51 corresponding to the albumin infusion bottle 11 in the tube through hole 20; the medical staff exert force on the drive auxiliary rod 23 and the drive rod 21, and the whole partition 19 moves upward, the elastic protective sleeve 22 is compressed and shortened, the second channel opening 62 of the liquid on-off tube 51 is located above the liquid level of the chamber 104, the liquid level floating body 53 falls from the second channel opening 62, and albumin enters the chamber 104 automatically and is injected into the patient.

After the albumin injection is completed, the liquid level in the chamber 104 drops, the second channel opening 62 corresponding to the normal saline is automatically reopened, and normal saline is automatically injected.

In the above operation process, first of all, compared with the traditional operation, the puncture device 12 is not required to be repeatedly inserted and removed, preventing pollution and having high safety; secondly, the medical staff only need to operate once to complete the action of changing the bottles for infusion, being convenient to operate.

In addition, the extension tube section 24 can be corrugated pipe structure, which can not only realize the extension function, but also can be fixed at the specified length position.

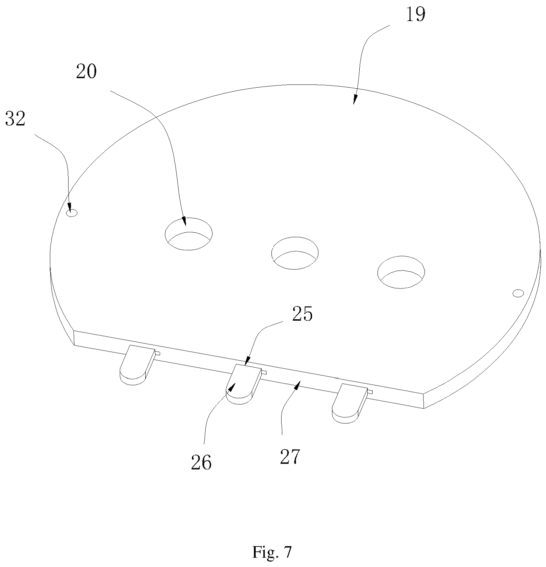

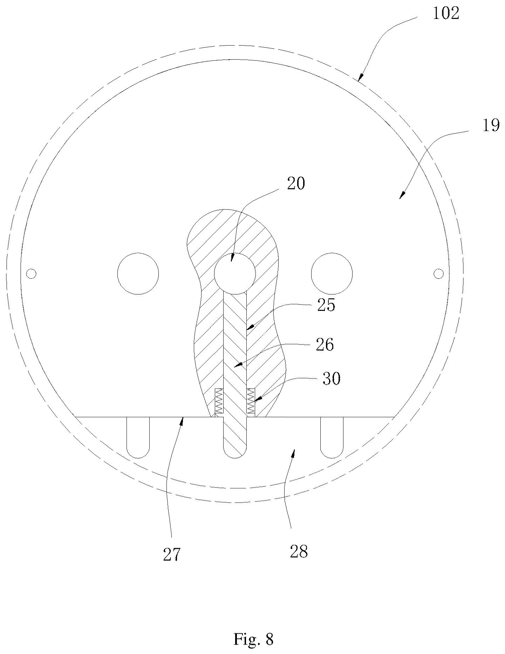

As shown in Fig. 7 and Fig. 8, a slot 27 is arranged on the outer edge of the partition 19, the pressing hole 25 is located on the wall of the slot 27, an interval space 28 is provided between the outer edge of the partition 19 of the slot 27 and the outer wall of the chamber 104, and the end of the pressing piece 26 is located in the interval space 28.

Therefore, on the one hand, the interval space 28 gives the main drip body 102 a certain deformation space, which is convenient for the medical staff to press; on the other hand, the contact area between the partition 19 and the inner wall of the chamber104 is reduced, and the movement smoothness of the partition 19 is improved.

As shown in Fig.6 and Fig.8, an elastic part 30 is connected between the pressing piece 26 and the pressing hole 25, and the elastic force of the elastic part 30 acts on the pressing piece 26, so that one end of the pressing piece 26 is located in the interval space 28, and the other end of the pressing 26 is separated from the outer wall of the liquid on-off tube 51.

Therefore, when the medical staff adjusts the length of the liquid on-off tube 51, the elastic part 30 can act on the pressing piece 26, and the end of the pressing piece 26 can be separated from the outer wall of the liquid on-off tube 51.

As shown in Fig. 6 and Fig. 7, a butting slot 32 is arranged on the side wall of the partition 19, and a butting block 31 is arranged on the first drip cap 101 and can be inserted into the butting slot 32.

Therefore, during the overall installation of the drip body 1, the butting block 31 on the first drip cap 101 can be inserted into the butting slot 32 on the partition 19, and then the partition 19 can be fixed on the first drip cap 101, so that the partition 19 can be installed in the chamber 104, and the main drip body 102 is inserted on the first drip cap 101.

### Embodiment 5:

Based on the use method of the infusion set in the embodiment 4, the operation steps are as follows:
Step 1: Mark the three puncture devices 12 as the first puncture device, the second puncture device and the third puncture device; the distances between the second channel openings 62 at the lower ends of the liquid on-off tubes 51 corresponding to the first puncture device, the second puncture device and the third puncture device and the second end 3 are from far to near in sequence;
   The control valves 18 corresponding to the first puncture device, the second puncture device and the third puncture device are the first valve, the second valve and the third valve respectively;
   Prepare three infusion bottles 11 respectively containing normal saline, albumin and other liquid, and insert the first puncture device, the second puncture device and the third puncture device into the infusion bottles 11 corresponding to normal saline, albumin and other liquid;
Step 2: Open the first valve, close the second valve and the third valve, and turn on the flow regulator 15;
Step 3: When the liquid level in the chamber 104 is higher than the second channel opening corresponding to the second puncture device 62 and the third channel opening corresponding to the third puncture device, close the first valve and turn off the flow regulator 15;
Step 4: Insert the infusion needle 16 into the vein of the patient;
Step 5: Turn on the flow regulator 15, and open the first valve, the second valve and the third valve;
Step 6: When the injection volume of normal saline corresponding to the first puncture device reaches the specified value, the medical staff can hold the outer side wall of the main drip body 102, the outer side wall of the main drip body 102 is deformed inward, the wall of the chamber 104 pushes the end of the pressing piece 26, the pressing piece 26 moves to the side of the tube through hole 20, and the end of the pressing piece 26 is butted with the liquid on-off tube 51 corresponding to the normal saline infusion bottle 11 in the tube through hole 20; the medical staff exert force on the drive auxiliary rod 23 and the drive rod 21, and the whole partition 19 moves downward and drives the liquid on-off tube 51 corresponding to the normal saline infusion bottle 11 to move downward, the elastic protective sleeve 22 is stretched, the second channel opening 62 of the liquid on-off tube 51 is located below the liquid level of the chamber 104, and the liquid level floating body 53 is blocked in the second channel opening 62, and the normal saline cannot continue to flow into the chamber 104;
   The medical staff hold the outer side wall of the main drip body 102 again, the outer side wall of the main drip body 102 is deformed inward, the wall of the chamber 104 pushes the end of another pressing piece 26, the pressing piece 26 moves to the side of the tube through hole 20, and the end of the pressing piece 26 is butted with the liquid on-off tube 51 corresponding to the albumin infusion bottle 11 in the tube through hole 20; the medical staff exert force on the drive auxiliary rod 23 and the drive rod 21, and the whole partition 19 moves upward, the elastic protective sleeve 22 is compressed and shortened, the second channel opening 62 of the liquid on-off tube 51 is located above the liquid level of the chamber 104, the liquid level floating body 53 falls from the second channel opening 62, and albumin enters the chamber 104 automatically;
Step 7: After the liquid of all infusion bottles 11 are injected, turn off the flow regulator 15.

In the above operation process, firstly, compared with the traditional operation, the puncture device 12 is not required to be repeatedly inserted and removed, preventing pollution and having high safety; secondly, the medical staff only need to operate once to complete the action of bottle changing and infusion, being convenient to operate; thirdly, in the case of a plurality of infusion bottles 11, the automatic bottle-by-bottle infusion effect can be achieved.

### Embodiment 6:

The difference from the above embodiments is that the liquid on-off tube 51 is preferably molded in one piece onto the frame 52 by injection. Of course, a separate design can also be adopted between the liquid on-off tube 51 and the frame body 52, and the liquid on-off tube 51 is inserted on the frame body 52.

A liquid channel 6 is arranged in the liquid on-off tube 51, a first channel opening 61 and a second channel opening 62 are respectively arranged at both ends of the liquid channel 6, the first channel opening 61 extends outside the frame body 52, and the second channel opening 62 extends inside the frame body 52.

The frame body 52 comprises a frame 521 and a frame cover 522, and the frame cover 522 is detachably connected to the frame 521.

The liquid level floating body 53 comprises a first floating body 40 and a second floating body 41, and the plug 34 is installed on the first floating body 40, and the first floating body 40 and the second floating body 41 are buckled mutually. A cavity 39 is formed between the first floating body 40 and the second floating body 41 buckled mutually.

The plug 34 is arranged on the first floating body 40 of the liquid level floating body 53, extends into the second channel opening 62 and can be blocked or separated from the second channel opening 62.

A sealing groove 35 is arranged on the inner frame wall of the frame body 52 and is located on one side close to the second channel opening 62; when the plug 34 is plugged inside the second channel opening 62, the side wall of the liquid level floating body 53 is butted in the sealing groove 35.

In addition, a guide head 36 is arranged on a second floating body 41 of the liquid level floating body 53, and a guide hole 37 is provided on the frame cover 522, and the guide head 36 is connected to the guide hole 37 in a slipping way; the guide head 36 and the plug 34 are symmetrically arranged.

A guide groove 38 is arranged on the inner frame wall of the frame body 52, and mutually slip fit is adopted between the side wall of the liquid level floating body 53 and the guide groove 38.

Of course, the embodiments are only typical examples of the invention. In addition, the invention can also have a variety of other embodiments. Any technical proposal formed by equivalent replacement or equivalent transformation falls within the scope of protection of the invention.

## Claims

1. A drip component, comprising a drip body (1), a chamber (104) is arranged inside the drip body (1), two opposite ends of the drip body (1) are respectively a first end part (2) and a second end (3), and a liquid outlet (4) is arranged on the drip body (1), and **characterized by** the following:
the first end (2) is provided with two or more liquid on-off parts (5);
the liquid on-off part (5) comprises a liquid channel (6), a first channel opening (61) and a second channel opening (62) are arranged at both ends of the liquid channel (6), the first channel opening (61) extends to the outside of the drip body (1), the second channel opening (62) is located in the chamber (104) and extends in the direction of the second end (3), and spacing between the second channel opening (62) and the second end (3) in each liquid on-off part (5) is different;
the liquid on-off part (5) also comprises a frame body (52) and a liquid level floating body (53), the frame body (52) is installed on the outside of the second channel opening (62), the frame body (52) is provided with a liquid inlet hole (7), and the liquid level floating body (53) is arranged in the frame body (52) and can be blocked or separated from the second channel opening (62).

2. The drip component according to claim 1, **characterized in that** a mounting hole (8) is arranged on the frame body (52), the second channel opening (62) is extended into the frame body (52) from the mounting hole (8) and is in a shape of flaring, and the flaring of the second channel opening (62) faces towards one side of the liquid level floating body (53).

3. The drip component according to claim 2, **characterized in that** the flaring size of the second channel opening (62) is larger than the pore size of the mounting hole (8) on the frame body (52).

4. The drip component according to claim 2, **characterized in that** the flaring outer edge of the second channel opening (62) is butted against the inner frame wall of the frame body (52).

5. The drip component according to any one of claims 1 to 4 , **characterized in that** the frame body (52) comprises a frame (521) and a frame cover (522), the mounting hole (8) is located on the frame (521), the liquid inlet hole (7) is located on the frame cover (522), a frame port (17) in the same direction as the second channel opening (62) is arranged on the frame (521), and the frame cover (522) is removably connected to the frame port (17) of the frame (521).

6. The drip component according to claim 1, **characterized in that** the drip body (1) comprises a first drip cap (101) and a main drip body (102), the first end (2) is located on the first drip cap (101), and the end part of the main drip body (102) is inserted on the side wall of the first drip cap (101).

7. The drip component according to claim 6, **characterized in that** two or more butting pieces (9) are arranged on the first drip cap (101), each butting piece (9) comprises an outer butt joint (91) and an inner butt joint (92), the outer butt joints (91) extend to the outside of the drip body (1), the inner butt joints (92) extend to the inside of the chamber (104), and the liquid channel (6) is located in the outer butt joint (91), the first drip cap (101) and the inner butt joint (92);
the liquid on-off part (5) comprises a liquid on-off tube (51), the end of the liquid on-off tube (51) is inserted into the inner butt joint (92), and the second channel opening (62) is located at the end of the liquid on-off tube (51) deviated from the inner butt joint (92).

8. The drip component according to claim 6, **characterized in that** the drip body (1) also comprises a second drip cap (103), the end of the main drip body (102) deviated from the first drip cap (101) is inserted on the side wall of the second drip cap (103), and the liquid outlet (4) is located on the second drip cap (103).

9. The drip component according to claim 7, **characterized in that** a clamping ring (10) is arranged on the outer side wall of the inner butt joint (92), and the liquid on-off tube (51) is clamped on the clamping ring (10).

10. An infusion set, comprising an infusion bottle (11), a puncture device (12), an infusion tube a (13), an infusion tube b (14), a flow regulator (15) and an infusion needle (16), and **characterized by** the following:
the infusion set also comprises the drip component in any of claims 1, 2, 3, 4, 6, 7, 8, 9, 10, 11, 12, 13, 14, 15, 16 and 17;
at least two infusion bottles (11), two puncture devices (12) and two infusion tubes a (13) are arranged, the infusion bottles (11), the puncture devices (12) and the infusion tubes a (13) correspond to the butting pieces (9) one by one, the puncture devices (12) are inserted into the corresponding infusion bottles (11), the infusion tube a (13) is connected between the puncture device (12) and the outer butt joint (91), the infusion tube b (14) is connected between the liquid outlet (4) and the infusion needle (16), and the flow regulator (15) is installed on the infusion tube b (14).
